# FASCICULE DE BREVET EUROPEEN

(11) **EP 2 959 511 B1**
(45) Date de publication et mention de la délivrance du brevet: **05.07.2017**
(21) Numéro de dépôt: 14708684.7
(22) Date de dépôt: 20.02.2014
(51) Int. Cl.: H01L 25/16, A61N 1/378, H01L 31/032, H01L 31/12, H02J 7/35

(54) **DISPOSITIF ET SYSTEME DE GENERATION PHOTOVOLTAIQUE ET SON APPLICATION A UN DISPOSITIF MEDICAL IMPLANTABLE.**
PHOTOVOLTAISCHE STROMERZEUGUNGSVORRICHTUNG UND SYSTEM UND ANWENDUNG DAVON AUF EINE IMPLANTIERBARE MEDIZINISCHE VORRICHTUNG
PHOTOVOLTAIC GENERATION DEVICE AND SYSTEM AND APPLICATION THEREOF TO AN IMPLANTABLE MEDICAL DEVICE

(30) Priorité: 25.02.2013 FR 1351640
(43) Date de publication de la demande: 30.12.2015
(73) Titulaire: Commissariat à l'Énergie Atomique et aux Énergies Alternatives, 75015 Paris (FR)
(72) Inventeur: KARST, Nicolas, F-57600 Folkling (FR); PERRAUD, Simon, F-83150 Bandol (FR)
(74) Mandataire: Gevers & Orès
(86) Numéro de dépôt international: PCT/IB2014/059128
(87) Numéro de publication internationale: WO 2014/128642

(56) Documents cités:
- WO-A1-2012/104503
- DE-A1- 4 435 602
- US-A1- 2006 085 051
- PUDOV A O ET AL: "CIGS J-V distortion in the absence of blue photons", THIN SOLID FILMS, ELSEVIER-SEQUOIA S.A. LAUSANNE, CH, vol. 480-481, 1 juin 2005 (2005-06-01), pages 273-278, XP027865238, ISSN: 0040-6090 [extrait le 2005-06-01] cité dans la demande

## Description

L'invention porte sur un dispositif et sur un système de génération photovoltaïque particulièrement adaptés pour des applications dans des dispositifs biomédicaux implantés, dans lesquelles l'éclairage s'effectue par voie transcutanée. L'invention porte également sur un système électronique comprenant un tel système de génération photovoltaïque et sur un dispositif médical implantable comprenant un tel système électronique.

Un dispositif, un système de génération et un système électronique selon l'invention peuvent également être utilisés dans d'autres applications que les dispositifs médicaux implantables.

On constate ces dernières années un intérêt croissant pour les dispositifs médicaux implantables, tels que les stimulateurs cardiaques, les neurostimulateurs, ou les systèmes permettant une détection et un contrôle biomédical *in vivo.* Dans un futur proche, les capteurs implantables pourront permettre par exemple un diagnostic précoce de certaines maladies ou encore de contrôler précisément le taux de sucre ou d'oxygène présent dans le sang en temps réel. Cependant, afin de fonctionner de façon autonome, les dispositifs médicaux implantables nécessitent une alimentation interne généralement assurée par une batterie primaire. Les batteries, dont les densités d'énergie sont relativement élevées (jusqu'à 300Wh/l), permettent d'alimenter des petits systèmes sur une durée allant de quelques jours à quelques années en fonction de la puissance nécessaire. Ainsi pour des dispositifs médicaux implantables, les densités de puissance des batteries les plus performantes actuellement disponibles obligent à un renouvellement régulier de ces dernières. Cependant, pour des applications *in vivo*, le remplacement des batteries n'est pas un acte anodin car il nécessite une intervention chirurgicale.

Pour éviter le remplacement des batteries primaires, il a été proposé d'utiliser des batteries dites « secondaires », rechargées par l'intermédiaire d'un dispositif de récupération d'énergie de type bobine (recharge par induction électromagnétique), transducteur ultrasonore (recharge par ultrasons) ou encore cellule photovoltaïque (recharge par lumière proche infrarouge). Ce type de dispositif présente l'avantage de ne pas nécessiter d'intervention chirurgicale pour remplacer une batterie déchargée. En effet, moyennant une recharge périodique des batteries secondaires par l'intermédiaire des dispositifs de récupération d'énergie citées précédemment, la durée de vie de ce type de dispositifs permet de s'approcher de celle des patients.

La recharge photovoltaïque constitue une approche particulièrement prometteuse, mais se heurte à des difficultés techniques importantes.

Dans leur article de 1999 «A wireless near-infrared energy system for medical implants » IEEE Enginnering Medecine Biology 18, 70 (1999), K. Murakawa *et al* ont proposé d'utiliser un laser proche infra-rouge couplé à une cellule photovoltaïque en silicium pour alimenter une batterie secondaire. Il s'agit d'implanter la cellule photovoltaïque et la batterie sous la peau et d'illuminer la cellule photovoltaïque par l'intermédiaire du laser proche infra-rouge, situé à l'extérieur du corps, lorsque la batterie doit être rechargée. En effet, la faible absorption du rayonnement proche infrarouge (780-1400 nm) par les tissus biologiques permet au rayonnement d'atteindre la cellule photovoltaïque implantée. Toutefois, les cellules photovoltaïques en silicium présentent une épaisseur relativement importante de l'ordre de 100µm ce qui les rend rigides et donc peu adaptées à l'implantation sous-cutanée. Les cellules photovoltaïques à couches minces, dont l'épaisseur peut être aussi faible que 10 µm et qui peuvent être réalisées sur un substrat souple, constituent une alternative plus prometteuse. Parmi les différents types de cellules photovoltaïques à couches minces, celles dites « de type CIGS » (acronyme de Cuivre - Indium - Gallium - Sélénium, car elles comprennent une couche d'absorption en un alliage de formule générale Culn₁₋ₓGaₓ(Se_{1-y},S_{y})₂, avec 0<x<1, 0≤y<1) sont particulièrement intéressantes en raison de leur efficacité élevée - comparable à celle des cellules en silicium et ce pour des épaisseurs nettement moins importantes.

Les cellules CIGS présentent cependant une caractéristique qui s'avère un inconvénient majeur pour une utilisation prévoyant un éclairage transcutané. En effet, il a été démontré que de telles cellules ne peuvent atteindre des performances électriques optimales que si le rayonnement les éclairant contient des photons d'énergie supérieure à la largeur de la bande interdite de leur couche tampon. Voir à ce propos l'article de A.Pudov et al., « CIGS J-V distortion in the absence of blue photons », Thin Solid Films 480 (2005), 273-278. En effet, si l'énergie des photons est supérieure à la largeur de la bande interdite de la couche tampon, une partie des photons est absorbée par la couche tampon créant une paire électron-trou, les trous photogénérés permettant de neutraliser les défauts de types accepteurs situés en surface de l'absorbeur, diminuant ainsi la barrière aux photoélectrons et permettant un alignement des bandes plus favorable.

Classiquement, les couches tampons utilisées dans ce genre de dispositifs sont des composés à base de Zinc [ZnO₁₋ₓSₓ], de Cadmium (CdS) ou d'Indium (In₂S₃) dont les largeurs de bandes interdites sont de 3,7eV, 2,8eV et 2,4eV respectivement. Ainsi, il est nécessaire d'éclairer ces cellules avec des photons de longueur d'onde inférieure à 335nm (ultraviolet), 443nm (bleu) et 516nm (vert) pour des cellules ayant respectivement comme couche tampon du Zn(O₁₋ₓ,Sₓ), de l'In₂S₃ et du CdS. Même si pour des longueurs d'onde dans le vert la transmission des tissus biologiques est plus importante que pour les longueurs d'onde dans le bleu ou l'ultra-violet, l'utilisation de couches tampon à base de Cd est inenvisageable dans des applications biomédicales implantées du fait de la très haute toxicité du Cd.

Il est donc difficile d'envisager d'avoir recours à des cellules photovoltaïques CIGS pour des applications implantables, car les photons dans l'ultra-violet ou le bleu nécessaires au bon fonctionnement de ces cellules sont absorbés par les tissus biologiques.

L'invention vise à surmonter cette difficulté et à permettre l'utilisation de cellules photovoltaïques de type «CIGS» dans des applications implantables, ou plus généralement en présence d'un éclairage dépourvu de photons d'énergie suffisamment élevée.

Les inventeurs sont partis du constat que, pour permettre un fonctionnement optimal des cellules photovoltaïques CIGS il n'est pas nécessaire que le flux de photons d'énergie supérieure à la largeur de bande interdite de la couche tampon soit continu. Une fois que les défauts de type accepteur ont été neutralisés, les cellules maintiennent, pendant une durée pouvant atteindre quelques jours, une efficacité de conversion élevée même si elles ne sont soumises qu'à un éclairage comprenant seulement des photons de plus basse énergie.

La solution procurée par la présente invention consiste à utiliser une diode électroluminescente émettant dans le vert, le bleu ou l'ultraviolet et transparente à des plus grandes longueurs d'onde, couplée à une cellule CIGS ou à un empilement de telles cellules. Les cellules CIGS, éclairées dans le rouge/infrarouge à travers la diode électroluminescente génèrent, avec une faible efficacité, une énergie électrique qui alimente la diode. A son tour, le rayonnement de « haute énergie » de la diode améliore progressivement l'efficacité des cellules ; lorsque cette efficacité atteint un niveau considéré comme suffisant, la diode est déconnectée et l'énergie électrique générée par effet photovoltaïque est utilisée pour alimenter une charge extérieure, par exemple pour recharger une batterie.

Ainsi un objet de l'invention est un dispositif photovoltaïque comprenant un module photovoltaïque de type CIGS présentant une surface, dite supérieure, destinée à être exposée à un rayonnement lumineux ; et une diode électroluminescente présentant une émission lumineuse à une longueur d'onde inférieure ou égale à 600 nm, de préférence inférieure ou égale à 443 nm et de manière encore plus préférée comprise entre 250 et 400 nm, et transparente au rayonnement dans le proche infrarouge, fixée à ladite surface supérieure du module photovoltaïque.

Selon différents modes de réalisation d'un tel dispositif :
- Ladite diode électroluminescente peut présenter une émission lumineuse à une longueur d'onde correspondant à une énergie photonique supérieure à une largeur de bande interdite d'une couche tampon dudit module photovoltaïque.
- Ledit module photovoltaïque de type CIGS peut être adapté pour générer un courant électrique lorsque sa surface supérieure est éclairée par un rayonnement proche infrarouge auquel ladite diode électroluminescente est transparente.
- Ledit module photovoltaïque peut comprendre une pluralité de cellules photovoltaïques connectées en série. En particulier, lesdites cellules photovoltaïques connectées en série peuvent être réalisées sous la forme d'un empilement de couches minces déposées sur un substrat commun.

Un autre objet de l'invention est un système de génération photovoltaïque comprenant un module photovoltaïque de type CIGS présentant une surface, dite supérieure, destinée à être exposée à un rayonnement lumineux ; et une diode électroluminescente présentant une émission lumineuse à une longueur d'onde inférieure à 600 nm, de préférence inférieure ou égale à 443 nm et de manière encore plus préférée comprise entre 250 et 400 nm, et transparente au rayonnement dans le proche infrarouge, agencée de façon à pouvoir éclairer ledit module photovoltaïque ; un système d'interrupteurs pour connecter sélectivement ledit module photovoltaïque à ladite diode électroluminescente ou à une borne d'alimentation d'une charge extérieure ; et un circuit de pilotage dudit système d'interrupteurs.

Avantageusement, ledit module photovoltaïque et ladite diode électroluminescente peuvent forment un dispositif photovoltaïque tel que décrit plus haut.

Ledit circuit de pilotage peut être adapté pour mesurer une puissance électrique générée par ledit module photovoltaïque et piloter ledit système d'interrupteur en fonction de la puissance mesurée. En particulier, ledit circuit de pilotage peut être adapté pour piloter ledit système d'interrupteur de telle sorte que ledit module photovoltaïque alimente ladite diode électroluminescente tant que ladite puissance mesurée n'excède par un seuil prédéfini, puis alimente une charge extérieure reliée à ladite borne d'alimentation.

Encore un autre objet de l'invention est un système électronique comprenant un système de génération photovoltaïque tel que décrit ci-dessus, une batterie connectée à ladite borne d'alimentation pour être rechargée par ledit système de génération photovoltaïque et au moins un circuit électronique connecté à ladite batterie pour être alimenté.

Encore un autre objet de l'invention est un dispositif médical implantable comprenant un tel système électronique. Dans ce cas, le circuit électronique peut être notamment un générateur d'impulsions de stimulation, un circuit de détection, acquisition, traitement et/ou de transmission de signaux acquis par un capteur implanté ou encore un circuit pilotant un dispositif implanté de délivrance de médicaments.

D'autres caractéristiques, détails et avantages de l'invention ressortiront à la lecture de la description faite en référence aux dessins annexés donnés à titre d'exemple et qui représentent, respectivement :
- la figure 1, une représentation schématique en coupe d'un dispositif photovoltaïque selon un mode de réalisation de l'invention ;
- la figure 2, une représentation schématique en coupe d'un dispositif photovoltaïque selon un mode de réalisation alternatif de l'invention ; et
- la figure 3 un diagramme fonctionne! d'un dispositif médical implantable selon un autre mode de réalisation de l'invention.

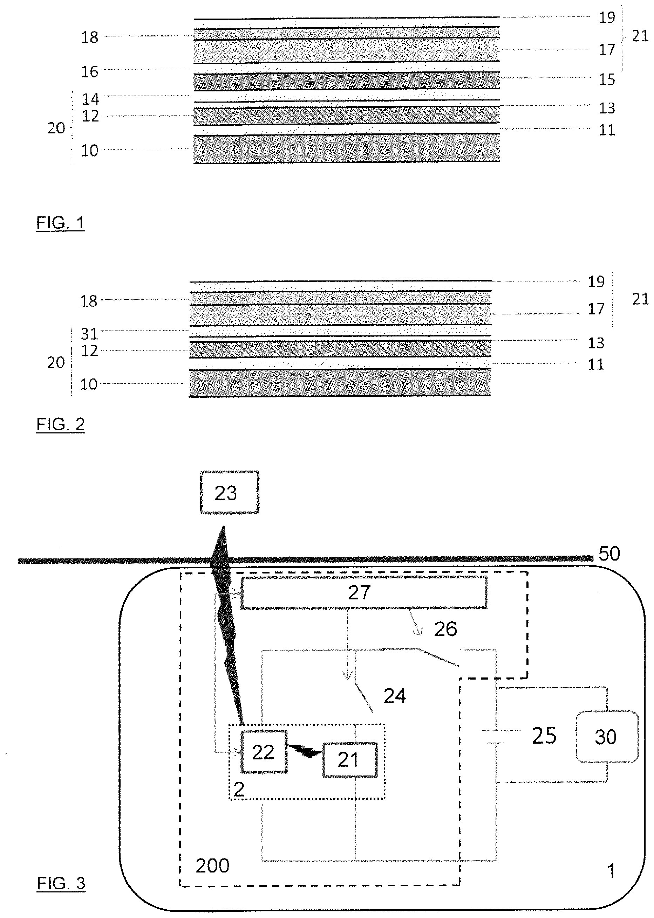

Sur la figure 1, la référence 10 indique un substrat souple ou rigide, qui peut être notamment en verre sodo-calcique, en polymère, en métal (acier, aluminium, titane,...). Sur le substrat 10, d'épaisseur comprise entre 10 µm et 5 mm préférentiellement comprise entre 0,5 mm et 2 mm, par exemple d'environ 1 mm, est déposée une électrode 11 en molybdène (Mo) dont l'épaisseur est comprise entre 100 nm et 2 µm, préférentiellement entre 200 nm et 1 µm, par exemple d'environ 500nm. Une couche 12 de CIGS [Cu(In₁₋ₓ,Gaₓ)(Se_{1-y},S_{y})₂, avec 0<x<1, 0≤y<1] semi-conducteur de type p, servant d'absorbeur de lumière, est déposée sur l'électrode 11 par coévaporation sous vide de matériaux élémentaires (Cu, In, Ga, Se) ou par recuit de sélénisation de précurseurs métalliques préalablement déposés. Les coefficients stoechiométriques x et y peuvent être très variables ; en effet, des rendements supérieurs à 19% ont été obtenu avec 0,69≤Cu/(In+Ga)≤0,98 et 0,21≤Ga/(In+Ga)≤0,38.

Afin de réaliser une jonction pn, une couche « tampon » 13 de ZnO₁₋ₓSₓ ou InO₁₋ₓSₓ (0<x<1), semi-conducteur de type n d'une épaisseur comprise ente 10 et 100 nm préférentiellement 50 nm est déposé par bain chimique, dépôt de couches atomiques (ALD), dépôt physique en phase vapeur (PVD), etc... sur la couche 12 de CIGS. Ensuite, une couche 14 d'un oxyde transparent conducteur est déposée sur la couche 13 afin de former une électrode transparente. II peut s'agir notamment d'une couche de ZnO dopé Al à 2% massique, présentant une épaisseur d'environ 500 nm. Il peut également s'agir d'une couche d'oxyde d'indium et étain (ITO) ou de SnO₂. On obtient ainsi une cellule photovoltaïque, indiquée sur la figure 1 par la référence 20. En réalité, il est généralement préférable de répéter une pluralité de fois l'empilement de couches 11 - 12 - 13 afin de réaliser un module photovoltaïque (référence 22 sur la figure 3) constitué par une connexion série de cellules élémentaires. Cela permet de générer une tension plus élevée, pouvant être utilisée directement pour alimenter la diode électroluminescente. Ainsi la mise en série de 10 cellules de tension comprise entre 0,5V - 0,6V (tension au point de puissance maximale typique des cellules CIGS) peut permettre d'atteindre des tensions de l'ordre de 5-6V. La densité de courant dépendra de la surface des cellules composant le module photovoltaïque et pourra aller de 30 mA pour des cellules de 1cm² à 300 mA pour des cellules de 10 cm².

La diode électroluminescente (DEL), indiquée par la référence 21, peut être fabriquée de la manière suivante. Une couche mince 17 d'un matériau semi-conducteur de type p est déposée sur un substrat 18 d'un matériau semi-conducteur de type n. Par exemple, une couche mince 17 de In₁₋ₓGaₓN de type p est déposée par épitaxie par faisceaux moléculaires (MBE) ou épitaxie en phase vapeur aux organométalliques (MOCVD) sur un substrat 18 de GaN monocristallin de type n. Ce type de DEL émet dans le bleu ou l'ultra-violet. D'autres couches semi-conductrices peuvent être ajoutées dans l'empilement formant la DEL 21 afin d'améliorer ses propriétés d'émissions comme expliqué dans l'article de K. Nakahara et al. « Improved External Efficiency InGaN-Based Light-Emitting Diodes with Transparent Conductive Ga-Doped ZnO as p-Electrodes », Japanese Journal of Applied Physics, Vol. 43, No. 2A, 2004, pp. L 180-L 182.

Le contact électrique du côté n (référence 19), peut être un plot, une grille métallique (par exemple un empilement Ti/Al/Ti/Au) ou même une couche d'un conducteur transparent tel que l'ITO. Le contact électrique du côté p (référence 16) est une électrode transparente qui peut être une couche mince d'un oxyde transparent conducteur à base de ZnO dopé Ga (ZnO:Ga), ou bien une couche ultra-mince métallique (Au, Cr...) d'épaisseur comprise entre 5 et 20 nm. Ce contact électrique 16 peut être complété par un plot ou une grille métallique (par exemple un empilement Ti/Au). La DEL 21 décrite ici est transparente au rayonnement proche infrarouge (780 - 1400 nm).

La DEL 21 peut être également fabriquée sur un substrat souple, par exemple par dépôt de couches minces semi-conductrices organiques sur substrat polymère, ou par des techniques de report de couches minces semi-conductrices inorganiques sur substrat polymère.

Le dispositif complet, identifié par la référence 2, est obtenu en fixant la DEL 21 au-dessus de la cellule photovoltaïque 20, c'est-à-dire sur sa surface « supérieure », opposée au substrat 10 et destinée à être éclairée. La fixation peut se faire, par exemple, par l'intermédiaire d'un film transparent et isolant 15, pouvant être constitué d'une résine époxy. Le film 15, d'épaisseur généralement comprise entre 50nm et 10µm, assure l'isolation électrique entre l'oxyde transparent conducteur 14 et l'électrode transparente 16, ainsi que la séparation physique entre la cellule photovoltaïque 20 et la DEL 21.

Dans un autre mode de réalisation, la cellule photovoltaïque et la DEL présentent une électrode transparente commune 31, ce qui permet de s'affranchir de la couche 15. Ce mode de réalisation est représenté sur la figure 2.

La figure 3 montre un schéma fonctionnel d'un dispositif médical implantable 1 comprenant un système de génération photovoltaïque 200 basé sur un dispositif photovoltaïque 2 tel que décrit ci-dessus.

Comme illustré sur cette figure, le système de génération photovoltaïque 200 utilise l'énergie lumineuse générée par une source externe 23 de rayonnement, émettant principalement dans le proche infrarouge, et transmise à travers la peau 50 pour charger une batterie 25 (par exemple, une micro-batterie solide à couches minces) qui, à son tour, alimente un circuit électronique 30 (par exemple, un générateur d'impulsions de stimulation).

Le système 200 comprend un dispositif photovoltaïque 2 du type illustré sur les figures 1 et 2, constitué par un module photovoltaïque 22 et une DEL 21, un système de commutation comprenant deux interrupteurs 24, 26 (réalisés, par exemple, par des transistors à effet de champ) et un circuit 27 de pilotage de ces interrupteurs, alimenté par le module 22 (ou, en variante, par la batterie 25). Le circuit de pilotage peut être basé sur un microprocesseur ou sur un circuit dédié, analogique, numérique ou hybride. Avantageusement, ce circuit mesure la puissance électrique générée par le module 22 et se sert du résultat de la mesure pour piloter les interrupteurs 24, 26 comme cela sera expliqué plus loin. Ces interrupteurs sont commandés en opposition de phase, c'est-à-dire que lorsque l'un est fermé, l'autre est ouvert. En particulier quand l'interrupteur 24 est fermé (ce qui correspond à la situation initiale, lors de la mise sous tension) le courant généré par le module 22 alimente la DEL 23, tandis que quand l'interrupteur 26 est fermé ce courant recharge la batterie 25 (plus généralement, alimente une charge externe).

Lorsqu'on commence à éclairer le module photovoltaïque 22 implanté à l'aide du rayonnement proche infrarouge émis par la source externe 23, ledit module fonctionne en mode dégradé, car l'éclairage ne contient pas de photons d'énergie suffisamment élevée pour permettre la neutralisation des défauts accepteurs, mais génère néanmoins une puissance suffisante pour alimenter la DEL 21 par l'intermédiaire de l'interrupteur 24 (tension 2-5V préférentiellement 3V, courant compris entre 5mA et 20mA préférentiellement 15mA, puissance comprise entre 10mW et 100mW préférentiellement 45mW). Ladite DEL génère alors un rayonnement lumineux dans la gamme de longueurs d'ondes absorbées par la couche tampon (typiquement 250nm-400nm préférentiellement environ 330nm pour une couche tampon en ZnOS). Au bout d'un certain temps d'exposition du module photovoltaïque 22 aux photons émis par la DEL 21, les performances électriques du module photovoltaïque 22 deviennent optimales. Cela est détecté par le circuit de pilotage 27 (par exemple par franchissement d'un premier seuil prédéterminé de puissance électrique générée), qui commande l'ouverture de l'interrupteur 24 et la fermeture de l'interrupteur 26. Cela entraine l'extinction de la DEL 21 ; la charge de la batterie secondaire 25 peut alors commencer. Inversement, le circuit de pilotage 27 peut commander le rallumage de la DEL 21 si la puissance générée par le module 22 décroit au-dessus d'un second seuil prédéterminé, inférieur audit premier seuil (hystérésis). D'autres logiques de commande sont toutefois envisageables, par exemple l'utilisation d'un simple minuteur.

En variante, le système photovoltaïque 200 peut comprendre un module photovoltaïque de type CIGS et une diode électroluminescente qui ne sont pas réunis dans un dispositif photovoltaïque tel que celui de la figure 1 ou de la figure 2, mais qui constituent des éléments discrets et distincts. Ces éléments devront alors être agencés de telle sorte que la diode électroluminescente puisse éclairer le module photovoltaïque, par exemple étant disposés face à face. Un tel mode de réalisation peut convenir en particulier pour des applications non implantables.

## Revendications

1. Dispositif photovoltaïque (2) comprenant :
- un module photovoltaïque (20) de type CIGS présentant une surface, dite supérieure, destinée à être exposée à un rayonnement lumineux ; et
- une diode électroluminescente (21) présentant une émission lumineuse à une longueur d'onde inférieure à 600 nm, de préférence inférieure ou égale à 443 nm et de manière encore plus préférée comprise entre 250 et 400 nm, et transparente au rayonnement dans le proche infrarouge, fixée à ladite surface supérieure du module photovoltaïque.

2. Dispositif photovoltaïque selon la revendication 1 dans lequel ladite diode électroluminescente présente une émission lumineuse à une longueur d'onde correspondant à une énergie photonique supérieure à une largeur de bande interdite d'une couche tampon (13) dudit module photovoltaïque.

3. Dispositif photovoltaïque selon l'une des revendications précédentes dans lequel ledit module photovoltaïque de type CIGS est adapté pour générer un courant électrique lorsque sa surface supérieure est éclairée par un rayonnement proche infrarouge auquel ladite diode électroluminescente est transparente.

4. Dispositif photovoltaïque selon l'une des revendications précédentes dans lequel ledit module photovoltaïque comprend une pluralité de cellules photovoltaïques connectées en série.

5. Dispositif photovoltaïque selon la revendication 4 dans lequel lesdites cellules photovoltaïques connectées en série sont réalisées sous la forme d'un empilement de couches minces déposées sur un substrat (10) commun.

6. Système de génération photovoltaïque (200) comprenant :
- un module photovoltaïque (20) de type CIGS présentant une surface, dite supérieure, destinée à être exposée à un rayonnement lumineux ; et
- une diode électroluminescente (21) présentant une émission lumineuse à une longueur d'onde inférieure à 600 nm, de préférence inférieure ou égale à 443 nm et de manière encore plus préférée comprise entre 250 et 400 nm, et transparente au rayonnement dans le proche infrarouge, agencée de façon à pouvoir éclairer ledit module photovoltaïque ;
- un système d'interrupteurs (24, 26) pour connecter sélectivement ledit module photovoltaïque à ladite diode électroluminescente ou à une borne d'alimentation d'une charge extérieure (25) ; et
- un circuit de pilotage (27) dudit système d'interrupteurs.

7. Système de génération photovoltaïque selon la revendication 6 dans lequel ledit module photovoltaïque et ladite diode électroluminescente forment un dispositif photovoltaïque (2) selon l'une des revendications 1 à 5.

8. Système de génération photovoltaïque selon l'une des revendications 6 ou 7 dans lequel ledit circuit de pilotage est adapté pour :
- mesurer une puissance électrique générée par ledit module photovoltaïque ; et
- piloter ledit système d'interrupteur en fonction de la puissance mesurée.

9. Système de génération photovoltaïque selon la revendication 8 dans lequel ledit circuit de pilotage est adapté pour piloter ledit système d'interrupteur de telle sorte que ledit module photovoltaïque alimente ladite diode électroluminescente tant que ladite puissance mesurée n'excède par un seuil prédéfini, puis alimente une charge extérieure reliée à ladite borne d'alimentation.

10. Système électronique comprenant :
- un système de génération photovoltaïque (200) selon l'une des revendications 6 à 9 ;
- une batterie (25) connectée à ladite borne d'alimentation pour être rechargée par ledit système de génération photovoltaïque ; et
- au moins un circuit électronique (30) connecté à ladite batterie pour être alimenté.

11. Dispositif médical implantable (1) comprenant un système électronique selon la revendication 10.

## Patentansprüche

1. Photovoltaische Vorrichtung (2), die Folgendes umfasst:
- ein photovoltaisches Modul (20) vom Typ CIGS, das eine sogenannte obere Fläche aufweist, die einer Lichtstrahlung ausgesetzt werden soll; und
- eine Leuchtdiode (21), die eine Lichtemission mit einer Wellenlänge von weniger als 600 nm, vorzugsweise kleiner oder gleich 443 nm und noch bevorzugter zwischen 250 und 400 nm aufweist, und die transparent für die Strahlung im nahen Infrarotbereich ist, die auf der oberen Fläche des photovoltaischen Moduls befestigt ist.

2. Photovoltaische Vorrichtung nach Anspruch 1, wobei die Leuchtdiode eine Lichtemission mit einer Wellenlänge entsprechend einer Photonenenergie aufweist, die größer als eine untersagte Bandbreite einer Pufferschicht (13) der photovoltaischen Vorrichtung ist.

3. Photovoltaische Vorrichtung nach einem der vorhergehenden Ansprüche, wobei das photovoltaische Modul vom Typ CIGS geeignet ist, einen elektrischen Strom zu erzeugen, wenn seine obere Fläche von einer Strahlung im nahen Infrarotbereich beleuchtet wird, gegen die die Leuchtdiode transparent ist.

4. Photovoltaische Vorrichtung nach einem der vorhergehenden Ansprüche, wobei das photovoltaische Modul eine Mehrzahl von in Serie geschalteten Photovoltaik-Zellen enthält.

5. Photovoltaische Vorrichtung nach Anspruch 4, wobei die in Serie geschalteten Photovoltaik-Zellen in Form eines Stapels dünner Schichten ausgeführt sind, die auf einem gemeinsamen Substrat (10) aufgebracht sind.

6. Photovoltaisches Stromerzeugungssystem (200), das Folgendes umfasst:
- ein photovoltaisches Modul (20) vom Typ CIGS, das eine sogenannte obere Fläche aufweist, die einer Lichtstrahlung ausgesetzt werden soll; und
- eine Leuchtdiode (21), die eine Lichtemission mit einer Wellenlänge von weniger als 600 nm, vorzugsweise kleiner oder gleich 443 nm und noch bevorzugter zwischen 250 und 400 nm aufweist, und die transparent für die Strahlung im nahen Infrarotbereich ist, die so gestaltet ist, dass sie das photovoltaische Modul beleuchten kann;
- ein System von Schaltern (24, 26) für den selektiven Anschluss des photovoltaischen Moduls an die Leuchtdiode oder an eine Einspeiseklemme einer externen Ladung (25); und
- eine Treiberschaltung (27) des Schaltersystems.

7. Photovoltaisches Stromerzeugungssystem nach Anspruch 6, wobei das photovoltaische Modul und die Leuchtdiode eine photovoltaische Vorrichtung (2) nach einem der Ansprüche 1 bis 5 bilden.

8. Photovoltaisches Stromerzeugungssystem nach einem der Ansprüche 6 oder 7, wobei die Treiberschaltung dazu geeignet ist:
- eine elektrische Leistung zu messen, die von photovoltaischem Modul erzeugt wird; und
- das Schaltsystem in Abhängigkeit von der gemessenen Leistung anzutreiben.

9. Photovoltaisches Stromerzeugungssystem nach Anspruch 8, wobei die Treiberschaltung zur Lenkung des Schaltersystems geeignet ist, so dass das photovoltaische Modul die Leuchtdiode versorgt, solange die gemessene Leistung einen vorgegebenen Schwellenwert nicht überschreitet, und dann eine externe Ladung versorgt, die an die Einspeiseklemme angeschlossen ist.

10. Elektronisches System, das Folgendes umfasst:
- ein photovoltaisches Stromerzeugungssystem (200) nach einem der Ansprüche 6 bis 9;
- eine Batterie (25), die an die Einspeiseklemme angeschlossen ist, um von photovoltaischen Stromerzeugungssystem geladen zu werden; und
- zumindest eine elektronische Schaltung (30), die an die Batterie angeschlossen ist, um versorgt zu werden.

11. Implantierbare medizinische Vorrichtung (1), die ein elektronisches System nach Anspruch 10 umfasst.

## Claims

1. A photovoltaic device (2) comprising:
- a CIGS photovoltaic module (20) having a surface, called the upper surface, intended to be exposed to luminous radiation; and
- a light-emitting diode (21) having a luminous emission at a wavelength shorter than 600 nm, preferably shorter than or equal to 443 nm and even more preferably comprised between 250 and 400 nm, and transparent to radiation in the near infrared, fastened to said upper surface of the photovoltaic module.

2. The photovoltaic device as claimed in claim 1, in which said light-emitting diode has a luminous emission at a wavelength corresponding to a photonic energy higher than a bandgap width of a buffer layer (13) of said photovoltaic module.

3. The photovoltaic device as claimed in one of the preceding claims, in which said CIGS photovoltaic module is suitable for generating an electrical current when its upper surface is illuminated by near infrared radiation to which said light-emitting diode is transparent.

4. The photovoltaic device as claimed in one of the preceding claims, in which said photovoltaic module comprises a plurality of photovoltaic cells connected in series.

5. The photovoltaic device as claimed in claim 4, in which said photovoltaic cells connected in series are produced in the form of a thin-film stack deposited on a common substrate (10).

6. A photovoltaic generation system (200) comprising:
- a CIGS photovoltaic module (20) having a surface, called the upper surface, intended to be exposed to luminous radiation; and
- a light-emitting diode (21) having a luminous emission at a wavelength shorter than 600 nm, preferably shorter than or equal to 443 nm and even more preferably comprised between 250 and 400 nm, and transparent to radiation in the near infrared, arranged so as to be able to illuminate said photovoltaic module;
- a system of switches (24, 26) for selectively connecting said photovoltaic module to said light-emitting diode or to a supply terminal of an exterior load (25); and
- a control circuit (27) for controlling said system of switches.

7. The photovoltaic generation system as claimed in claim 6, in which said photovoltaic module and said light-emitting diode form a photovoltaic device (2) as claimed in one of claims 1 to 5.

8. The photovoltaic generation system as claimed in one of claims 6 or 7, in which said control circuit is suitable for:
- measuring an electrical power generated by said photovoltaic module; and
- controlling said switching system depending on the measured power.

9. The photovoltaic generation system as claimed in claim 8, in which said control circuit is suitable for controlling said switching system such that said photovoltaic module powers said light-emitting diode when said measured power is below a preset threshold, then powers an exterior load connected to said supply terminal.

10. An electronic system comprising:
- a photovoltaic generation system (200) as claimed in one of claims 6 to 9;
- a battery (25) connected to said supply terminal so as to be recharged by said photovoltaic generation system; and
- at least one electronic circuit (30) connected to said battery so as to be powered.

11. An implantable medical device (1) comprising an electronic system as claimed in claim 10.
